Europäisches Patentamt

European Patent Office

Office européen des brevets

(11) Publication number: **0 285 511 B1**

# EUROPEAN PATENT SPECIFICATION

(45) Date of publication of patent specification: **25.11.92**    (51) Int. Cl.⁵: **G01N 33/577**, G01N 33/86, C12P 21/00, C12N 5/00

(21) Application number: **88400761.8**

(22) Date of filing: **29.03.88**

The file contains technical information submitted after the application was filed and not included in this specification

(54) **Immunoassay of thrombomodulin.**

(30) Priority: **01.04.87 JP 77507/87**

(43) Date of publication of application:
**05.10.88 Bulletin 88/40**

(45) Publication of the grant of the patent:
**25.11.92 Bulletin 92/48**

(84) Designated Contracting States:
**DE FR GB IT**

(56) References cited:
**EP-A- 0 253 331**
**WO-A-87/00050**

**JOURNAL OF BIOLOGICAL CHEMISTRY, vol. 260, no. 29, 15 December 1985,The American Society of Biological Chemists Inc. (US); I.MARUYAMA et al., pp. 15432-15438**

**BIOCHEMISTRY, vol. 26, no. 14, 14 July 1987, American Chemical Society (US); D.WEN et al., pp. 4350-4357**

**CHEMICAL ABSTRACTS, vol. 105, 1986, Columbus,OH (US); p. 400, no. 168482u**

(73) Proprietor: **MITSUBISHI GAS CHEMICAL COMPANY, INC.**
**5-2, Marunouchi 2-chome Chiyoda-Ku Tokyo, 100(JP)**

(72) Inventor: **Tsubouchi, Jiro**
**8-52, Minamisasaguchi 1-chome Niigata-shi Niigata 944(JP)**
Inventor: **Kazama, Mutsuyoshi**
**4-26-4, Zenpukuji Suginami-ku Tokyo 167(JP)**
Inventor: **Ishii, Hidemi**
**Honmachida Jutaku Ro-834, 19, honmachida Machida-shi Tokyo 194(JP)**
Inventor: **Nakano, Masahiko**
**4-11-5, Oomiyadai Chiba-shi Chiba 280(JP)**

(74) Representative: **Bourgognon, Jean-Marie et al Cabinet Flechner 22, Avenue de Friedland F-75008 Paris(FR)**

THE JOURNAL OF BIOLOGICAL CHEMISTRY, vol. 259, no. 19, 10 October1985, The American Society of Biological Chemists Inc. (US); H.H.SALEM et al., pp. 12246-12251

BIOLOGICAL ABSTRACTS, vol. 80, 1985, Biological AbstractsInc., Philadelphia, PA (US); K.SUZUKI et al., no. 4928

J. CLIN. INV., vol. 76, 1985; ISHIL et al., pp. 2178-2181

**Description**

FIELD OF THE INVENTION

The present invention relates to a method of immuno assay for human thrombomodulin using a monoclonal antibodies binding specifically to human thrombomodulin, to a novel monoclonal antibodies binding to human thrombomodulin, which can be used for the said immuno assay, and to hybridomas producing the said antibody.

With the monoclonal antibodies of the present invention, the analysis of the function and structure of human thrombomodulin has become possible. In the field of basic medical and clinical medical sciences, it is very important to determine the concentrations of thrombomodulin in blood or urine with monoclonal antibodies against thrombomodulin to understand the behavior of thrombomodulin.

BACKGROUND OF THE INVENTION

Thrombomodulin has been found as a protein which exists on the surface of vascular endothelial cells, acting as a receptor of thrombin, and which significantly accelerates the activation of Protein C by thrombin [Esmon, C.T., & Owen, W. G.: Proc. Natl. Acad. Sci. U.S.A. 78, 2249-2252 (1981)].

It has been known that, upon formation of thrombin-thrombomodulin complex, thrombin loses its properties as a coagulation factor such as its abilities to activate blood coagulation factors V and VIII, to convert fibrinogen to fibrin and to activate plateletes [Esmon, C.T., et al,: J. Biol, Chem. 257, 7944-7947 (1982)., ibid., 258, 12238-12242 (1983)].

It has also been known that Protein C is activated by thrombin-thrombomodulin complex and that the activated Protein C shows potent anti-coagulation effect through inactivation of activated blood coagulation factors V (FVa) and VIII (FVIIIa) [Suzumi, K., et al.: J. Biol. Chem. 258, 1914-1920 (1983), Vehar, G.A. & Davie, E.M.: Biochemistry 19, 401-410 (1980)] and promotes fibrinolysis by releasing tissue plasminogen activator (tPA) from the wall of blood vessel [Comp, P.C., & Esmon, C.T.: J. Clin. Invest. 68, 1221-1228 (1981)].

It has been reported that thrombomodulin directly inhibits blood coagulation factor Xa which converts prothrombin to thrombin [Thrompson, E.A., & Salem, H.H.: J.Clin. Invest. 78, 13-17 (1986)].

Thrombomodulin isolated from human placenta is a glycoprotein with a molecular weight of 105,000 as measured in the presence of a reducing agent, or 75,000 in the absence of it. Thrombomodulin exists on endothelial cells of the artery, vein, capillary vessel, lymph duct, and, in particular, abundantly in the lung and placenta. In placenta, thrombomodulin is found on syncytiotrophoblasts where the embryo and fetus receive nutrition from the maternal blood, and where the blood tends to pool. These facts suggest a possibility that thrombomodulin plays an important role as an anticoagulant. On the other hand , thrombomodulin has not been found in the brain tissue, and thrombi are likely to form in the brain. These facts attract interests of people.

Recently, the radio immuno assay (hereinafter referred to as RIA) analysis with antiserum against human thrombomodulin has revealed that thrombomodulin exists in the circulating blood and urine (Ishii, H., & Majerus, P.W.: J. Clin. Invest. 76, 2178-2181 (1985)]. Although the origins of thrombomodulin in blood and urine and its roles in controlling the coagulation in the circulating blood are not known, there is a possibility that it regulates the formation of thrombi as an anticoagulation factor. If we could elucidate the acting mechanism of thrombomodulin in the blood and understand the behavior of thrombomodulin in the blood by measuring the concentration of thrombomodulin in the blood, it would be very significant in the field of basic and clinical medical sciences.

As mentioned previously, the measurement of thrombomodulin in blood by RIA with antiserum against human thrombomodulin has been reported. This method, however, suffers from certain defects. For example, it is quite difficult to stably obtain antisera having a certain level of affinity to human thrombomodulin. Since the antiserum is often contaminated with other antibodies recognizing other proteins than thrombomodulin, there is a possibility that the measurement of thrombomodulin could not be successfully performed due to such contamination. Moreover, the measuring procedure of this method is not simple nor easy.

Recently, monoclonal antibodies have widely been used for the analysis of functions and structures of antigenic proteins and for immuno assay because of their advantages that they are specific to a single antigen determinant and that antibodies having the same and singly specificity can stably be produced. Especially, for the analysis of the function and molecule of an antigenic protein, a finding of an antibody recognizing the part affecting the function of the protein would be an efficient step.

With respect to human thrombomodulin, there has so far been only one report by I. Maruyama et al. [J. Biol. Chem., 260, 15432-15438, (1985)] that a monoclonal antibody (IgG-TM) binding to human thrombomodulin is produced by a hybridoma prepared by cell fusion of myeloma cell line Sp2/0-Ag14 and spleen cells of Balb/C mouse previously immunized with human thrombomodulin from placenta. IgG-TM is a monoclonal antibody which binds to the thrombin binding site of thrombomodulin, by binding to which the antibody inhibits the binding of thrombin to human thrombomodulin. There has not yet been developed any method for measuring human thrombomodulin in blood and urine using a monoclonal antibody. It is neither clear nor obvious from the above mentioned publication whether or not the said monoclonal antibody can be used for immuno assay for human thrombomodulin. Moreover, two or more types of monoclonal antibodies each of which binds to a different antigen determinant are required in the immuno assay by the so-called sandwich method using monoclonal antibodies, which is often used for measurement of small amounts of antigenic proteins.

Under the circumstances, development of a monoclonal antibody having a high affinity and specificity to human thrombomodulin, which can be employed for elucidation of the function and structure of human thrombomodulin playing an important role as a regulator of the blood coagulation and fibrinolysis and for immuno assay for human thrombomodulin in blood or urine, and a hybridoma capable of stably producing the said monoclonal antibody have been wanted. Further, development of a method for measuring therombomodulin in a sample with a high sensitivity and specificity by an easy measuring procedures has long been desired in the fields of the basic medical and clinical medical sciences.

## BRIEF EXPLANATION OF THE DRAWINGS

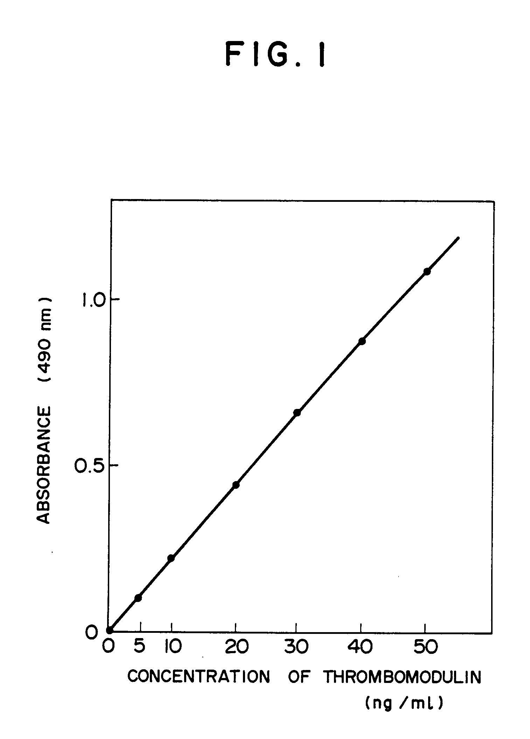

Figures 1 through 6 show the relations between the concentrations of human thrombomodulin in samples and the absorbances measured in the Examples.

## SUMMARY OF THE INVENTION

The present invention is directed to the uses of monoclonal antibodies binding specifically to human thrombomodulin in a sandwich immuno assay method for human thrombomodulin, by which human thrombomodulin can be measured with a high sensitivity and an easy procedure.

## DETAILED EXPLANATION OF THE INVENTION

The hybridomas can be produced by the method of Köhler and Milstein (Köhler, G., & Milstein, C.: Nature, 256, 495-497 (1975) . Namely, after a mammal is immunized with human thrombomodulin, spleen cells harvested from the mammal are fused with myeloma cell line to yield a hybridoma. The hybridoma producing a monoclonal antibody binding to human thrombomodulin can be selected by, for example, subjecting supernatants of culture media of the resulting hybridomas to enzyme immuno assay (EIA) with microplates on which human thrombomodulin is fixed.

The human thrombomodulin used as an antigen can be isolated from human placenta and purified according to the method of Salem et al. (Salem, H.H., et al.: J. Biol. Chem. 259, 12246-12251 (1984) .

The mammal used for immunization with human thrombomodulin is not particularly limited, but should be selected taking into consideration to the compatibility with myeloma used for fusion. Generally speaking, mice and rats are used for this purpose.

The immunization can be effected in accordance with a conventional method. For example, human thrombomodulin is diluted with physiological saline solution or the like to an adequate concentration and made up to a suspension with complete Freund's adjuvant, which is administered to an animal by intraperitoneal injections or the like. Usually 10-100 $\mu$g of protein is given to an animal one to several times every 2-4 weeks. The final immunization is usually conducted by an intravenous injection of physiological saline solution containing 10-100 $\mu$g of protein. It is preferable to harvest spleen cells 3-4 days after the final immunization. As for myeloma, known myeloma cell lines such as mouse cell lines SP-2, NS-1 and P3-UI and rat cell line Y3.Ag 1.2.3 can be employed. For the cell-fusion, a fusing promoting agent such as polyethylene glycol of an average molecular weight of 1,000-6,000 or Sendai virus (HVJ) is used. With respect to the ratio of the spleen cells to the myelomas, usually 1 to 10 spleen cells per 1 myeloma cell are used.

The isolation of the desired hybridoma is carried out by culturing the fused cells obtained as above in a selection medium usually used for hybridoma selection. The myeloma used for the cell-fusion is defective in hypoxanthine-guanine phosphoribosyl transferase (HGPRT), and therefore can not survive in HAT medium (hypoxanthine, aminopterin and thymidine are contained), but the hybridoma can. Therefore the desired hybridoma can be obtained by selecting the cells survived in the culturing in HAT medium.

The screening and cloning of the hybridoma producing the objective monoclonal antibody can be performed in accordance with a conventional limited dilution method. The hybridoma obtained as above can readily be subcultured and stored for a long time in liquid nitrogen.

The thus obtained hybridoma can be grown in a culture medium or in the peritoneal cavity of mammalia. The monoclonal antibody produced can be isolated and purified from a supernatant of a culture medium of the hybridoma or the ascites or serum of the mammalia transplanted hybridoma as above. Generally, centrifugation, dialysis, salt out with ammonium sulfate, column chromatography on DEAE-cellulose, gel filtration, affinity chromatography and the like are used for this purpose.

The monoclonal antibody binding to human thrombomodulin obtained as above has a strong binding activity against human thrombomodulin. In the monoclonal antibodies used in the method of the present invention, various types of monoclonal antibodies binding to different antigen determinants of thrombomodulin are included. It has become possible to measure a very small amount of human thrombomodulin with a high specificity and high sensitivity by EIA, RIA or fluoro sandwich type immuno assay(FIA) using those antibodies.

For example, a known enzyme immuno assay such as the one disclosed in "KOUSOMENEKI SOKUTEIHOU" (The 2nd ed., Ishikawa, Eiji, IGAKUSHOIN, 1982) can be used. In the following, the EIA with sandwich method is explained. Monoclonal antibody labelled with radio isotope or a fluorescent material can equally be used in these methods. In the sandwich method, an antigen (standard or sample) is added to an antibody (the first antibody) fixed on a solid support. As the result of the antigen-antibody reaction, the antigen is bound to the antibody fixed on the solid support. To the antigen fixed on the solid support, the second antibody labelled with enzyme is added, which causes an antigen-antibody reaction, The second antibody labelled with an enzyme is bound to the antigen fixed on the solid support. After the second antibody labelled with an enzyme which is not bound to the antigen is removed, a substrate is added to cause an enzymatic reaction. For the measurement of antigens, a calibration curve is prepared by determining the levels of the enzymatic reactions using known amounts of antigens. With this calibration curve, the amounts of antigens in the sample can be determined. In order to simplify the procedure, an antigen and an enzyme-labelled antibody are added at the same time to an antibody fixed onto a solid support (one step sandwich method). Usually, the first antibody and second antibody binding each other to different parts of the antigen are used in combination. Each of the first antibody and second antibody may be the same type of antibody or a mixture of different types of antibodies.

In the sandwich method of the present invention, fragments of antibody having an antigen-binding activity such as fragment $F(ab')_2$ prepared by digestion of an antibody with pepsin, fragment Fab' obtained by reduction of fragment $F(ab')_2$ or fragment Fab prepared by digestion with papain or their labelled fragments can be used as antibody or labelled antibody.

As an enzyme to be bound to the antibodies, peroxidase, $\beta$-D-galactosidase, alkaline phosphatase, glucose oxidase and the like can be used. As for the method for labelling antibodies, known methods such as those disclosed in "TANKURON KOUTAI" (Iwasaki, Tatsuo, et al., KODANSHA SCIENTIFIC, 1984) or in "KOUSOMENEKI SOKUTEIHO" (The 2nd ed., Ishikawa, Eiji, et al. IGAKUSHOIN, 1982) are used to prepare an enzyme-labelled antibody.

As for solid supports, sticks, beads, microplates or tubes made of silicone, nylon, plastics or glass can be employed.

The present invention will more precisely be illustrated by the following non-limiting representative examples.

Example 1

Preparation of monoclonal antibody binding to human thrombonodulin:

(1) Preparation of the antigen used for immunization:

In accordance with the above-mentioned method of Salem et al., 1.4 mg of human thrombomodulin was obtained from two human placentas.

(2) Preparation of spleen cells of the immunyzed mice:

An emulsion of 20 $\mu$g of human thrombomodulin and complete Freund's adjuvant was intraperitoneally injected to each of 3 male Balb/c mice. Physiological saline solution containing 20 $\mu$g of human thrombomodulin was intravenously injected to the mice 3 weeks after the intraperitoneal injection. All the mice were sacrificed 3 days after the final immunization and the spleens were excised and used for cell fusion.

(3) Preparation of hybridoma:

The spleens were spread to pieces with tweezers. The spleen cells were suspended in RPMI-1640 medium (RPMI-1640) supplemented with 0.29 g/l of L-glutamine, 0.11 g/l of puruvic acid, 0.06 g/l of kanamycin sulfate, 0.06 g/l of crystalline penicillin G.K and 1.2 g/l of $NaHCO_3$, and passed through a nylon mesh to obtain a suspension of single cells.

The red blood cells in the suspension were destroyed by the treatment with a mixture of 0.83 % ammonium chloride solution and 0.17 M Tris(hydroxymethyl)-aminomethane hydrochloric acid buffer (PH 7.6) (9:1 by volume) for 2 minutes at 4° C and removed by centrifugation.

Mouse myeloma cell line SP-2 grown to logarithmic phase in RPMI-1640 supplemented with 10 % fetal calf serum (hereinafter referred to as 10 % FCS-RPMI-1640) were washed twice with RPMI-1640.

The spleen cells and the mouse myeloma cells were suspended at a cell ratio of 5 : 1 in RPMI-1640 and centrifuged to remove the medium. After the cells were heated to 37° C on a water bath, and 1.3 ml of 50 % aqueous solution of polyethylene glycol of an average molecular weight of 1500 (produced by Boeringer Manheim Yamanouchi), which was kept to 37° C, was gradually added to the cells in one minute and incubated for 1 minute.

To the fused incubated mixture, 9 ml of RPMI-1940 was gradually added in 4 minutes to terminate the cell fusion. After removal of supernatant by centrifugation, the cells were resuspended in 10 % FCS-RPMI-1640 at a concentration of 5 x $10^6$ cells, 100 $\mu$l of the suspension was poured into each well of a plate (96 wells) (Corning Glass Works). After the plate was incubated for 1 day at 37° C under an atmosphere of 5.5 % $CO_2$, 100 $\mu$l of 10% FCS-RPMI-1640 containing aminopterine (4 x $10^{-4}$ M), thymidine (1.6 x $10^{-5}$ M) and hypoxanthine (1 x $10^{-4}$ M) (hereinafter, HAT medium) was added to each of the wells. One day after, a half of the medium was removed by aspiration and 100 $\mu$l of HAT medium was added to each of the wells. Then, incubation was contained while a half of the medium was replaced with fresh HAT medium every 2 or 3 days. Ten days after, the growth of hybridoma was observed.

(4) Selection of hybridoma producing monoclonal antibodies binding to human thrombomodulin:

Screening for monoclonal antibodies bound to human thrombomodulin is supernatant of hybridoma culture was carried out with EIA by a human thrombomodulin-fixed microplate.

The fixing of human thrombomodulin onto a 96 well immunomicroplate (produced by Nunc Products) was carried out by adding 50 $\mu$l of 0.2 $\mu$g/ml of human thrombomodulin PBS (0.01 M phosphate buffer, PH 7.4, 0.15 M NaCl) solution to each of the wells and allowing to absorb at room temperature for 2 hours. After washing the plate three times with PBS, PBS containing 0.2 % bovine serum albumin (hereinafter, BSA-PBS) was added to the plate to completely block the wells to prevent non-specific adsorption of protein. After addition of 50 $\mu$l each of supernatant of the hybridoma culture obtained as above to the wells, the wells were incubated for 1 hour at room temperature. The assay of the antibody bound to the well was carried out with VECTASTAIN ABC Kit (Vector Laboratories). Namely, after the plate was washed 3 times with PBS containing 0.05 % Tween® 20 (hereinafter, Tween20-PBS), 50 $\mu$l each of biotinylated goat anti-mouse immunoglobulin antiserum was added to each of the wells and incubated for 1 hour. After washing with Tween20-PBS, 50 $\mu$l of avidinylated peroxidase was added to each of the wells and incubated for 20 minutes. After washing 3 times with Tween20-PBS, 100 $\mu$l of the mixture of 0.03 % $H_2O_2$ and 100 mM citrate buffer solution (PH 5:0) containing 0.8 mg/ml of o-phenylenediamine dihydrochloride (1 : 1 by volum) was added to each of the wells and incubated at room temperature. The enzymatic reaction was terminated by addition of 100 $\mu$l of 2N sulfuric acid and the absorbance of the reaction mixture was measured at 490 nm with Immuno Reader NJ-2000 (Inter Med).

In 592 wells out of 768 wells to which the fused cells were distributed, growing hybridomas were observed, and antibody binding to human thrombomodulin was detected in 126 wells. Cloning of hybridoma was first started with 16 wells which were found strong in the antibody activity. The cells in 20 wells of the remaining positive wells were kept frozen and thawed at a later day for cloning.

(5) Cloning for hybridoma producing monoclonal antibodies binding to human thrombomodulin:

The culture of hybridoma producing monoclonal antibodies binding to human thrombomodulin were transferred to a 24 wells-flat bottom microplate (Corning Glass Works) using Balb/c mouse thymocytes (1 x $10^7$ cells/ml) as feeder layers and cultured.

The hybridomas produced were cloned by limited dilution method with 96 well-microplate using Balb/C mouse thymocytes (1 x $10^7$ cells/ml) as feeder layers. Repeating the cloning procedure twice, total 17 clones were obtained.

(6) Purification of monoclonal antibody binding to human thrombomodulin:

The hybridoma (2-5 x $10^6$ cells) obtained in the above item (5) was transplanted in peritoneal cavity of Balb/C mouse which was given 0.5 ml each of Pristane (Aldrich Chemical) 10 and 3 days before transplantation. After one week, ascites of the mouse was harvested and monoclonal antibody was isolated from the ascites with 50 % saturated ammonium sulfate solution. The ammonium sulfate precipitate residue dissolved in a small amount of PBS was also purified with Affi-Gel Protein A-MAPS Kit (Bio-Rad Laboratories). Finally, the antibody was dialyzed against PBS and stored at 4°C or in frozen. Purified antibody was obtained in a yield of 0.5-10 mg per 1 ml of ascites.

Example 2

Characteristics of the monoclonal antibodies binding to human thrombomodulin

(1) Determination of immunoglobulin subclass of the monoclonal antibodies:

The immunoglobulin subclass of the purified antibody was determined by Ouchterlony test with class-specific goat antimouse immunoglobulin antisera (Meroy Laboratories). The results are shown in Table 1.

## Table 1

| Antibody | Immunoglobulin subclass |
|----------|-------------------------|
| TMmAb2 | $IgG_1$ |
| TMmAb3 | $IgG_{2b}$ |
| TMmAb5 | $IgG_1$ |
| TMmAb7 | $IgG_1$ |
| TMmAb8 | $IgG_1$ |
| TMmAb9 | $IgM$ |
| TMmAb10 | $IgG_1$ |
| TMmAb11 | $IgG_1$ |
| TMmAb12 | $IgG_1$ |
| TMmAb15 | $IgG_1$ |
| TMmAb20 | $IgG_1$ |
| TMmAb24 | $IgG_{2b}$ |
| TMmAb26 | $IgM$ |
| TMmAb27 | $IgM$ |
| TMmAb28 | $IgG_1$ |
| TMmAb32 | $IgM$ |
| TMmAb33 | $IgG_1$ |

(2) Determination of a difference of human thrombomodulin binding sites of the monoclonal antibodies:

In order to determine whether the human thrombomodulin binding sites of the monoclonal antibodies are the same as or different from others, each of the monoclonal antibodies was labelled with horse-radish peroxidase (hereinafter HRP) and subjected to competition reaction with the unlabelled monoclonal antibodies toward human thrombomodulin and the occurrence of the inhibition of the binding to the human thrombomodulin was observed. The labelling of the antibodies with HRP was carried out by periodic acid oxidation method ("TANKURON KOUTAI", Iwasaki, Tatsuo, et al., KODANSHA SCIENTIFIC, 1984). Namely, 4 mg of HRP (TOYOBO) was dissolved in 1 ml of distilled water and 200 $\mu$l of 0.1 M sodium periodate was added to the solution, which was then allowed to react at room temperature for 20 minutes. The solution was dialyzed overnight against 1 mM sodium acetate buffer solution (pH 4.4) at 4°C. To the resulting solution was added 20 $\mu$l of 0.2 M sodium carbonate to adjust to pH 9.0. Then, 1 ml of 0.01 M sodium carbonate buffer solution (pH 9.5) containing 5 mg/ml of the antibody added immediately to the solution and stirred at room temperature for 2 hours. After cooling with ice, 0.1 ml of $NaBH_4$ was added to the solution and allowed to react at 4°C for 2 hours. The reaction mixture was dialyzed against PBS at 4°C overnight and subjected to gel filtration with Superose 12 column (pharmacia Fine Chemicals) equilibrated with PBS to isolate the unreacting HRP and antibody from the labelled antibody. The labelled antibody fraction was selected by measuring the absorbance at 280 nm and 403 nm. All the antibodies were labelled and retained the binding activity, to human thrombomodulin.

The competition reaction was carried out as follows:

After 100 $\mu$l of PBS solution of human thrombomodulin (0.2 $\mu$g/ml) was added to each well of immunomicroplate and incubated for 2 hours at room temperature, thereby the thrombomodulin was adsorbed in the wells. The wells were washed three times with PBS and BSA-PBS was added to the wells to prevent non-specific adsorption of proteins into the wells. A mixture predetermined amount of the HRP-labelled antibody and the unlabelled antibody (0-1 mg/ml) serially diluted with Tween20-PBS containing 0.2 % BSA (hereinafter, BSA-Tween20-PBS) were added to each of the wells. After 1 hour antigen-antibody reaction, the plate was washed four times with Tween20-PBS. To each of the wells, 100 $\mu$l of a substrate (0.015 % $H_2O_2$ and 50 mM citrate buffer solution containing 0.4 mg/ml of o-phenylenediamine dihydrochloride) was added and subjected to enzymatic reaction at room temperature. The reaction was stopped by addition of 100 $\mu$l of 2 N sulfuric acid to each of the wells. The absorbance of the reaction mixture was measured at 490 nm with Immuno Reader-NJ-2000. The results are shown Tables 2-1 and 2-2.

Table 2-1

| Antibody | Enzyme-labelled antibody | | | | | |
|---|---|---|---|---|---|---|
| | TMmAb7 | TMmAb8 | TMmAb11 | TMmAb20 | TMmAb28 | TMmAb33 |
| TMmAb7 | + | - | + | - | ± | - |
| TMmAb8 | ± | + | ± | - | - | + |
| TMmAb11 | + | - | + | - | ± | - |
| TMmAb20 | - | - | - | + | + | - |
| TMmAb24 | - | - | - | ± | + | - |
| TMmAb28 | - | - | - | + | + | - |
| TMmAb33 | ± | + | ± | - | - | + |
| + : Inhibition | | | | | | |
| ± : Slight inhibition | | | | | | |
| - : No inhibition | | | | | | |

Table 2-1

| Antibody | Enzyme-labelled antibody | | | | | |
|---|---|---|---|---|---|---|
| | TMmAb2 | TMmAb8 | TMmAb11 | TMmAb12 | TMmAb15 | TMmAb20 |
| TMmAb2 | + | − | − | + | + | − |
| TMmAb3 | − | + | − | − | − | − |
| TMmAb8 | − | + | − | − | − | − |
| TMmAb10 | − | − | − | − | − | + |
| TMmAb12 | + | − | − | + | − | − |
| TMmAb15 | + | − | − | − | + | − |
| TMmAb20 | − | − | − | − | − | + |

+ : Inhibition

− : No inhibition

From the above results, the monoclonal antibodies can be divided into the groups (TMmAb7, TMmAb11), (TMmAb3, TMmAb8, TMmAb33), (TMmAb10, TMmAb20, TMmAb24, TMmAb28) and (TMmAb2, TMmAb12, TMmAb15). The results indicate that the antigen binding sites of the monoclonal antibodies of one group are different from those of the monoclonal antibodies belonging to the other groups, and that the monoclonal antibodies belonging to the same group recognize the same or very close antigen determinants.

(3) Inhibitory activity of the monoclonal antibody against Protein C activation by human thrombomodulin:

The inhibitory activities of the monoclonal antibodies against Protein C activation by thrombin-thrombomodulin complex were measured as follows:

A mixture of 5 µl of the purified monoclonal antibody (200 ng), 10 µl of human thrombomodulin (0.43 pmole) and 70 µl of 20 mM Tris-HCl buffer solution (pH 7.4) containing 0.5 % BSA, 1.35 mM $CaCl_2$ and 0.1 M NaCl was incubated for 15 minutes at room temperature. After addition of 5 µl of bovine thrombin (0.45 µM) and 10 µl of Protein C (2.13 µM), the reaction mixture was incubated for 30 minutes at 37°C. By addition of 150 µl of hirudin (1.7 U/ml) to the mixture, the thrombin was inactivated. To the mixture was added 250 µl of a synthetic fluorescent substrate (100 µM Boc-Leu-Ser-Thr-Arg-MCA, Peptide Laboratories), which was then incubated for 10 minutes at 37°C. The fluorescence emitted from the hydrolysis of the substrate by the activated Protein C was measured by a spectrofluorometer (Hitachi 650-10 type) (excitation: 380 nm, luminescence: 460 nm). The inhibitory percentage of the Protein C activation by the monoclonal antibodies were determined, based upon the Protein C activation without monoclonal antibodies as 100 %.

The results are shown in Table 3.

Table 3

| Antibody | Inhibition of Protein C Activation (%) |
|---|---|
| TMmAb2 | 0 |
| TMmAb7 | 0 |
| TMmAb8 | 85.0 |
| TMmAb11 | 3.3 |
| TMmAb12 | 2.8 |
| TMmAb15 | 0 |
| TMmAb20 | 89.7 |
| TMmAb24 | 13.0 |
| TMmAb28 | 54.5 |
| TMmAb33 | 86.8 |

Among the examined ten monoclonal antibodies, the monoclonal antibodies TMmAb8, TMmAb20 and TMmAb33 strongly inhibited Protein C activation under the reaction conditions of the above examples. TMmAb2, TMmAb7, TMmAb11, TMmAb12, and TMmAb15 showed little or no inhibitory activity against Protein C activation.

(4) Inhibition of formation of thrombin-thrombomodulin complex by the monoclonal antibody:

The inhibitory activity of the monoclonal antibody against formation of thrombin-thrombomodulin complex was measured by subjecting thrombin and the monoclonal antibody to competitive reaction toward human thrombomodulin.

The purified monoclonal antibody and purified bovine thrombin (MOCHIDA PHARMACEUTICAL) were mixed in BSA-Tween20-PBS in amounts of 20 ng/ml and 1.7 $\mu$g/ml, respectively. The amount of the monoclonal antibody bound to human thrombomodulin were measured by EIA using 96 wells immunomicroplate onto which human thrombomodulin is fixed in the same manner as Example 2-(2).

The results are given in Table 4, which were calculated in reference to the absorbance of a control using no thrombin as 100 %.

## Table 4

| Antibody | Amounts of antibody bound (%) |
|----------|-------------------------------|
| TMmAb2   | 102.0 |
| TMmAb7   | 97.8  |
| TMmAb8   | 92.9  |
| TMmAb11  | 97.4  |
| TMmAb12  | 96.8  |
| TMmAb15  | 97.5  |
| TMmAb20  | 17.7  |
| TMmAb24  | 31.9  |
| TMmAb28  | 8.4   |
| TMmAb33  | 89.2  |

Among the 10 tested monoclonal antibodies, the bindings to human thrombomodulin of the monoclonal antibodies, TMmAb20, TMmAb24 and TMmAb28 were significantly inhibited by the competition by thrombin, but the others are hardly affected.

It is clear from the results of Example 2-(3) and (4) using some of our monoclonal antibodies that the monoclonal antibodies having at least the following characteristics were prepared: 1) being the antibody binding to the thrombin binding site or its vicinity of human thrombomodulin to inhibit the binding of thrombin to human thrombomodulin, which in turn inhibits Protein C activation. 2) being the antibody recognizing the human thrombomodulin antigen determinants where an antibody binding does not inhibit the binding of thrombin to human thrombomodulin, and not inhibiting Protein C activation by thrombin-thrombomodulin complex. 3) being the antibody binding to the part of the antigen to which antibody binding causes inhibition of Protein C activation by thrombin-thrombomodulin complex but not thrombin binding to human thrombomodulin.

The monoclonal antibody prepared by I. Maruyama et al. is regarded to belong to the above 1) group in our classification. In this 1) group of antibodies, TMmAb20, TMmAb24 and TMmAb28 are included. But, they are different from each others in immunogloblin subclass or in the inhibitory activity. The monoclonal antibodies belonging to the above 2) and 3) groups are all novel monoclonal antibodies and recognize antigen determinants of human thrombomodulin to which the antibody binds without inhibition of thrombin-binding to the human thrombomodulin.

Example 3

Measurement of human thrombomodulin using the monoclonal antibodies binding to human thrombomodulin

(1) EIA by sandwich method:

Generally in sandwich method in which monoclonal antibodies are used as the first antibody fixed onto a solid support) and second antibody (enzyme-labelled antibody), it is inadequate to use the same monoclonal antibodies or a combination of monoclonal antibodies binding to similar antigen binding sites.

From the results of the experiments of the competition reaction of the monoclonal antibodies in Example 2-(2), usable combinations can be determined.

In the following, there will be illustrated representative examples in which TMmAb20 as the first antibody and HRP-labelled TMmAb11 as the second antibody are used, and as the first antibody TMmAb20 and as the second antibody HRP-labelled Fab′ fragment of TMmAb2 are used.

In addition to these combinations, human thrombomodulin can be measured with other combinations of the monoclonal antibodies of the present invention, though they are not specifically exemplified.

(a) Two step sandwich EIA:

TMmAb20 was prepared at concentration of 5 $\mu$g/ml with PBS, and 100 $\mu$l of it was added to each of the wells of an immuno microplate, which were then incubated for 2 hours at room temperature for adsorption. The plate was washed three times with PBS and subjected to blocking reaction with BSA-PBS for 1 hour at room temperature to prevent non-specific adsorption. After addition of 100 $\mu$l of purified human thrombomodulin diluted with BSA-Tween20-PBS to each of the wells, the plate was incubated for 1 hour at room temperature. The plate was washed three times with Tween20-PBS, and TMmAb11 labelled with HRP by the procedure set forth in Example 2-(2) was added to the plate, which were then incubated for 1 hour at room temperature. After washing the plate three times with Tween20-PBS, 100 $\mu$l of the substrate (0.015 % $H_2O_2$ 50 mM citrate buffer solution (pH 5.0) containing 0.4 mg/ml of o-phenylenediamine dihydrochloride) was added to each of the wells and incubated for 5 minutes at room temperature. The reaction was stopped by addition of 100 $\mu$l of 2N $H_2SO_4$ to each of the wells. The absorbances at 490 nm were measured with Immuno Reader NJ-2000.

The results are given in Figure 1.

From Figure 1, it is clear that a linear relationship exists between the concentration of thrombomodulin and the absorbances. With this calibration curve, the concentrations of thrombomodulin in plasma and 24 hour-excreted urine of healthy human beings were determined. The results are shown in Tables 5 and 6.

Table 5

| Sample | Concentration of thrombomodulin in plasma (ng/ml) |
|---|---|
| N.M. | 22.3 |
| S.T. | 30.5 |
| N.O. | 28.8 |
| H.S. | 30.6 |
| S.U. | 24.7 |
| Y.S. | 22.1 |
| H.I. | 23.0 |
| N.H. | 46.8 |
| J.T. | 23.9 |
| M.O. | 25.2 |

Table 6

| Sample | Concentration of thrombomodulin in urine (ng/ml) |
|---|---|
| H.U. | 212 |
| M.N. | 103 |
| N.H. | 205 |
| T.Y. | 159 |
| S.T. | 104 |
| H.S. | 51 |

(b) One step sandwich EIA:

An example of one step sandwich method for immuno assay for human thrombomodulin wherein, as the first antibody fixed on a solid support, TMmAb20 and, as the second antibody, HRP-labelled fragment Fab′ of TMmAb2 prepared by digestion with pepsin (HRP-labelled Fab′) were used is given in the following.

(Preparation of HRP-labelled Fab′)

10 ml of PBS solution of TMmAb2 (2 mg/ml) was adjusted to pH 4.0 by addition of 1 M citrate buffer solution (pH 3.2). After addition of 250 μg of pepsin derived from porcine stomach mucosa (Sigma Chemical Company) to the monoclonal antibody solution, the solution was incubated for 12 hours at 37°C to digest the antibody. The solution was adjusted to pH 7.0 by addition of 3M Tris-HCl buffer solution (pH 8.6) and concentrated to 2 ml, which was then applied to Superose 12HR 16/50 column (Pharmacia Fine Chemicals) equilibrated with 0.1 M phosphate buffer solution (pH 6.0) to afford F(ab')$_2$ fraction. Thus, 5.5 mg of F(ab')$_2$ was recovered. The F(ab')$_2$ fraction was concentrated to 2 ml and 2-mercapatoethylamine and EDTA were added to the concentrated fraction to final concentrations of 10 mM and 0.5 mM, respectively. The mixture was then incubated for 90 minutes at 37°C to reduce F(ab')$_2$. The reaction mixture was applied to Superose 12 HR 16/50 column equilibrated with 0.1 M phosphate buffer solution (pH 6.0) conataining 5 mM EDTA to give Fab' fraction. Thus, 4.8 mg of Fab' was recovered.

After 10 mg of HRP was dissolved in 0.1 M phosphate buffer solution (pH 7.0), 150 $\mu$l of N,N-dimethylformamide solution of N-($\epsilon$-maleimidocaproyloxy)succinimide (10 mg of N-($\epsilon$-maleimidocaproxyloxy)succinimide was dissolved in 1 ml of N,N-dimethylformamide) was added to the solution and incubated for 60 minutes at 30°C. The reaction mixture was applied to PD 10 column (Pharmacia Fine Chemicals) equilibrated with 0.1 M phosphate buffer solution (pH 6.0) to afford HRP fraction.

Thus, a maleimido group was introduced to HRP. After 1.1 ml of maleimido-introduced HRP (3.6 mg/ml) was added to 3 ml of the above Fab' solution (1.5 mg/ml). The mixture was incubated for 60 minute at 30°C. The reaction mixture was concentrated to 2 ml and applied to Superose 12HR 16/50 column equilibrated with 0.1 M phosphate buffer solution (pH 6.5) to give 12 ml of HRP-labelled Fab' fraction.

The molar ratio of HRP to Fab' of the resulting HRP-labelled Fab' was about 1 : 1.

(One step sandwich enzyme immuno assay

TMmAb20 was prepared at concentration of 10 $\mu$g/ml with 50 mM carbonate buffer solution (pH 9.5) and 200 $\mu$l of it was added to each of the wells of an immunomicroplate. The plate was incubated at room temperature for 2 hours for adsorption of the antibody and washed three times with PBS.

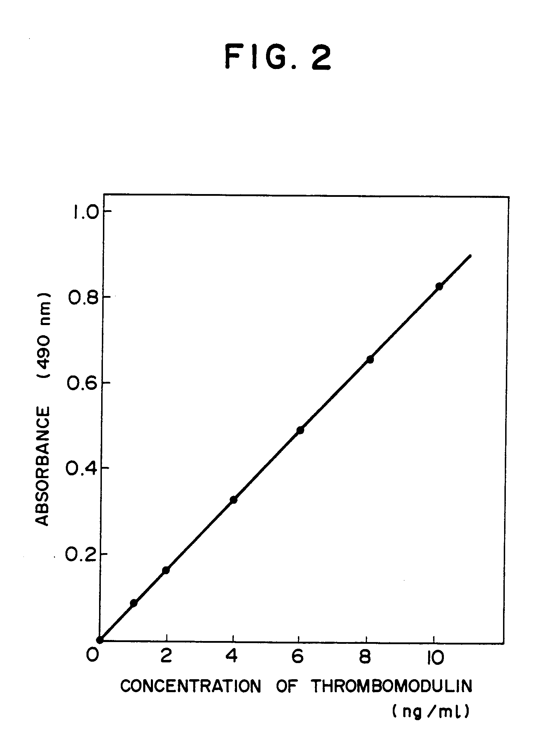

After filled with BSA-PBS, the wells were incubated at room temperature for blocking. Thus, a TMmAb20-fixed plate was prepared. The HRP-labelled Fab' of TMmAb2 prepared as above was 200 fold-diluted with Bsa-Tween20-PBS, 150 $\mu$l of which was then added to each of the wells. To each of the wells, the purified human thrombomodulin diluted with BSA-Tween20-PBS was added and allowed to stand for 1 hour at room temperature. After washing three times with Tween20-PBS, 200 $\mu$l of the substrate solution (0.015 % $H_2O_2$, 50 mM citrate buffer (pH 5.0) containing 0.4 mg/ml of o-phenylenediamine dihydrochloride) was added to each of the wells and incubated at room temperature for 20 minutes. The ezymatic reaction was stopped by addition of 100 $\mu$l of 2N $H_2SO_4$ to each of the wells. The absorbances at 490 nm of the reaction mixtures were measured with Immuno Reader NJ-2000. The relationship between the concentrations of human thrombomodulin and the absorbances at 490 nm is shown in Figure 2.

By the development of the novel monoclonal antibodies binding to human thrombomodulin and the hybridomas stably producing the said monoclonal antibodies, there has now been developed an enzyme immuno assay by which a small amount of human thrombomodulin in blood or urine samples can be measured.

## Claims

1. The use of a monoclonal antibody which is able to bind to human thrombomodulin as an antigen said human thrombomodulin bound to the monoclonal antibody being further able to bind to thrombin, in a sandwich immuno-assay of human thrombomodulin.

2. The use of a monoclonal antibody according to claim 1 recognizable to human thrombomodulin, which may be produced by a hybridoma prepared by a method comprising
   A) cell-fusion of human thrombomodulin-immunized mouse spleen cells and mouse myeloma cells defective in hypoxanthine-guanine phosphoribosyl transferase, said cell-fusion being performed in the presence of polyethylene glycol or Sendai virus,
   B) selection of desired hybridoma by selecting the cells survived in the culturing in hypoxanthine, aminopterin and thymidine-contained medium (HAT medium), and
   C) selection of the desired hybridoma by a competition reaction test of a monoclonal antibody thereof with unlabelled monoclonal antibodies toward human thrombomodulin, an inhibitory activity test of the monoclonal activity thereof against protein C activation or an inhibition test of thrombin-thrombomodulin complex by the monoclonal antibody thereof in a sandwich immuno-assay of human thrombomodulin.

3. An immuno assay method of human thrombomodulin in blood or urine which comprises contacting with human thrombomodulin at least two monoclonal antibodies which are able to recognize and bind to different sites, respectively, of the human thrombomodulin wherein said monoclonal antibodies are selected from the group consisting of

1) an antibody bindable to thrombin binding site or vicinity thereof of human thrombomodulin to inhibit binding of thrombin to human thrombomodulin,

2) an antibody recognizing the human thrombomodulin antigen determinants where an antibody binding does not inhibit the binding of thrombin to human thrombomodulin and not inhibiting Protein C activation by thrombin-thrombomodulin complex, and

3) an antibody bindable to the part of antigen to which antibody binding causes inhibition of Protein C activation by thrombin-thrombomodulin complex but not thrombin binding to human thrombomodulin.

4. A method according to claim 3, wherein the said antibody is fragment F(ab')$_2$ Fab or Fab'.

5. A method according to claim 3, wherein a sandwich method is carried out by use of (1) an antibody bound to an insoluble carrier and (2) a labelled antibody.

6. A method according to claim 5 wherein said insoluble carrier is a plastic vessel, plastic beads, latex beads, glass beads or metal particles.

7. A method according to claim 5, wherein the labelled antibody is an antibody labelled with an enzyme, a radio isotope or a fluorescent material.

**Patentansprüche**

1. Verwendung eines monoklonalen Antikörpers, der sich als Antigen an Human-Thrombomodulin zu binden vermag, wobei das an den monoklonalen Antikörper gebundene Human-Thrombomodulin Thrombin zu binden in der Lage ist, in einem Sandwich-Immunoassay auf Human-Thrombomodulin.

2. Verwendung eines Human-Thrombomodulin erkennenden monoklonalen Antikörpers gemäß Anspruch 1, der mittels eines Hybridoms erzeugt werden kann, das auf folgende Weise erhältlich ist:

A) Zellverschmelzung von mit Human-Thrombomodulin immunisierten Maus-Milzzellen mit Maus-Myelomzellen, denen Hypoxanthin-Guanin-Phosphoribosyl-Transferase mangelt, wobei die Zellverschmelzung in Gegenwart von Poly(ethylenglycol) oder dem Sendai-Virus durchgeführt wird;

B) Selektion der gewünschten Hybridome durch Auswahl der die Kultivierung in einem Hypoxanthin, Aminopterin und Thymidin enthaltenen Medium (HAT-Medium) überlebenden Zellen; und

C) Selektion der gewünschten Hybridome mittels eines kompetitiven Bindungstests eines ihrer monoklonalen Antikörper mit unmarkierten monoklonalen Antikörpern auf Human-Thrombomodulin, mittels eines Hemmaktivitätstests auf monoklonale Antikörper-Aktivität derselben gegen Protein C-Aktivierung oder mittels eines Hemmungstests des Thrombin-Thrombomodulinkomplexes durch den monoklonalen Antikörper derselben in einem Sandwich-Immunoassay auf Human-Thrombomodulin.

3. Immunoassay-Verfahren auf Human-Thrombomodulin im Blut oder Urin, das umfaßt:

Kontaktierung von Human-Thrombomodulin mit mindestens 2 Typen von monoklonalen Antikörpern, die in der Lage sind, verschiedene Regionen des Human-Thrombomodulins zu erkennen und sich an diese anzukoppeln, wobei die monoklonalen Antikörper ausgewählt sind aus der Gruppe, die umfaßt:

1) einen Antikörpertyp, der an die Thrombinbindende Region oder deren Nachbarregion des Human-Thrombomodulins anzukoppeln in der Lage ist, um die Thrombinankopplung an Human-Thrombomodulin zu hemmen;

2) einen Antikörpertyp, der die Human-Thrombomodulin-Antigendeterminanten zu erkennen vermag, wobei eine Antikörperankopplung weder die Thrombinbindung an Human-Thrombomodulin noch die Protein C-Aktivierung durch den Thrombin-Thrombomodulin-Komplex hemmt; und

3) einen Antikörper, der an die Antigenregion anzukoppeln ist, an der die Antikörperbindung die Hemmung der Protein C-Aktivierung durch den Thrombin-Thrombomodulinkomplex, nicht aber die Hemmung der Thrombinbindung an Human-Thrombomodulin bewirkt.

4. Verfahren nach Anspruch 3, wobei als Antikörper das Fragment F(ab')$_2$,, Fab oder Fab' eingesetzt wird.

**5.** Verfahren nach Anspruch 3, wobei ein Sandwichverfahren unter Verwendung von einem
(1) an einem unlöslichen Träger fixierten Antikörpertyp und
(2) einem markierten Antikörpertyp zur Anwendung kommt.

**6.** Verfahren nach Anspruch 5, wobei als unlöslicher Träger ein Kunststoffgefäß, Kunstoff-, Latex- oder Glaskügelchen bzw. Metallteilchen eingesetzt werden.

**7.** Verfahren nach Anspruch 5, wobei als markierter Antikörpertyp ein mit einem Enzym, einem radioaktiven Isotop oder einem fluoreszierenden Stoff markierter eingesetzt wird.

**Revendications**

**1.** L'utilisation d'un anticorps monoclonal qui est apte à se fixer à de la thrombomoduline humaine comme antigène, la thrombomoduline humaine, fixée à l'anticorps monoclonal, étant en outre apte à se fixer à de la thrombine, dans un dosage immunologique sandwich de la thrombomoduline humaine.

**2.** Utilisation d'un anticorps monoclonal suivant la revendication 1 reconnaissable à la thrombomoduline humaine, qui peut être produit par un hybridome préparé par un procédé comprenant
A) une fusion cellulaire de cellules spléniques de la souris immunisée à la thrombomoduline humaine et de cellules de myélome de souris déficientes en hypoxanthineguanine phosphoribosyl transférase, cette fusion cellulaire étant effectuée en la présence de polyéthylèneglycol ou de virus de Sendai,
B) une sélection de l'hybridome désiré en sélectionnant les cellules qui survivent lors de la culture dans un milieu contenant de l'hypoxanthine, de l'aminoptérine et de la thymidine (milieu HAT), et
C) une sélection de l'hybridome désiré par un essai de réaction par compétition de l'un de leurs anticorps monoclonaux et d'anticorps monoclonaux non marqués, sur la thrombomoduline humaine, par un essai d'inhibition de son activité monoclonale sur l'activation de la protéine C ou par un essai d'inhibition du complexe thrombine-thrombomoduline par son anticorps monoclonal, dans un dosage immunologique sandwich de la thrombomoduline humaine.

**3.** Procédé de dosage immunologique de thrombomoduline humaine du sang ou de l'urine, qui consiste à mettre de la thrombomoduline humaine en contact avec au moins deux anticorps monoclonaux qui sont susceptibles de reconnaître des sites différents respectivement de la thrombomoduline humaine et de s'y fixer, les anticorps monoclonaux étant choisis parmi
1) un anticorps susceptible de se fixer à un site de la thrombomoduline humaine de fixation de la thrombine ou à proximité de ce site, pour inhiber la fixation de la thrombine à la thrombomoduline humaine,
2) un anticorps reconnaissant les déterminants d'antigène de la thrombomoduline humaine où une fixation d'un anticorps n'inhibe pas la fixation de la thrombine à la thrombomoduline humaine et n'inhibant pas l'activation de la protéine C par le complexe thrombine-thrombomoduline, et
3) un anticorps susceptible de se fixer à la partie d'un antigène à laquelle une fixation d'un anticorps provoque l'inhibition de l'activation de la protéine C par un complexe thrombine-thrombomoduline, mais non la fixation de la thrombine à la thrombomoduline humaine.

**4.** Procédé suivant la revendication 3, dans lequel l'anticorps est le fragment F(ab')$_2$ Fab ou Fab'.

**5.** Procédé suivant la revendication 3, dans lequel on effectue un procédé sandwich en utilisant (1) un anticorps fixé à un support insoluble et (2) un anticorps marqué.

**6.** Procédé suivant la revendication 5, dans lequel le support insoluble est un récipient en matière plastique, des billes en matière plastique, des billes en latex, des billes de verre ou des particules métalliques.

**7.** Procédé suivant la revendication 5, dans lequel l'anticorps marqué est un anticorps marqué par une enzyme, par un radio-isotope ou par une substance fluorescente.

16

# F I G. I

# FIG. 2